# FASCICULE DE BREVET EUROPEEN

(11) **EP 2 691 125 B1**
(45) Date de publication et mention de la délivrance du brevet: **24.05.2017**
(21) Numéro de dépôt: 12718428.1
(22) Date de dépôt: 22.03.2012
(51) Int. Cl.: A61M 1/36

(54) **CAPUCHON AMÉLIORÉ POUR PIÈGE À BULLES**
VERBESSERTER DECKEL FÜR EINE BLASENFALLE
IMPROVED CAP FOR A BUBBLE TRAP

(30) Priorité: 31.03.2011 TN 11154
(43) Date de publication de la demande: 05.02.2014
(73) Titulaire: BOU AKEZ, Naoufel, 2092 El Manar 1 Tunis (TN)
(72) Inventeur: BOU AKEZ, Naoufel, 2092 El Manar 1 Tunis (TN)
(74) Mandataire: Regimbeau
(86) Numéro de dépôt international: PCT/TN2012/000001
(87) Numéro de publication internationale: WO 2012/134409

(56) Documents cités:
- WO-A1-2006/054957
- US-A- 5 591 251
- US-A1- 2006 173 395

## Description

### DOMAINE TECHNIQUE GENERAL.

La présente invention concerne le domaine des dispositifs extracorporels d'hémodialyse par ligne artérielle et ligne veineuse, et plus particulièrement les composants de piège à bulles veineux ou de chambres d'expansion artérielles utilisés dans de tels dispositifs.

Elle concerne en particulier les parties supérieures respectives d'un piège à bulles veineux ou d'une chambre d'expansion artérielles, qui peuvent êtres fabriquées en forme de capuchons ou intégrées directement dans la forme des chambres du piège à bulles veineux ou de la chambre d'expansion artérielle respectivement.

### ETAT DE L'ART

Les circuits extracorporels d'hémodialyse conventionnels sont fréquemment confrontés à des problèmes dus à la formation de caillots dans la circulation extracorporelle d'hémodialyse. Ces caillots obstruent les différentes pièces formant le circuit ou limitent le flux en circulation, nécessitant alors d'arrêter la séance d'hémodialyse

Afin de remédier à ces inconvénients, la demande de brevet WO 2006/054957 au nom du demandeur présente un circuit extracorporel d'hémodialyse amélioré, comprenant des moyens d'extraction des caillots sanguins se formant dans le circuit.

Ce document présente un circuit extracorporel d'hémodialyse tel qu'illustré sur la figure 1.
Dans ce circuit extracorporel d'hémodialyse 1, le sang est prélevé d'un patient P, entre par une ligne artérielle 2, et est entrainé par une pompe 3 au travers d'une chambre d'expansion artérielle 4, d'un dialyseur 5, d'un piège à bulles veineux 6, d'une ligne veineuse 7 et d'une ligne de recirculation 8.
La ligne veineuse 7 assure le retour du sang vers le patient P.
Le piège à bulles veineux 6 comprend un orifice d'alimentation 61, un orifice de pression 62, un orifice d'évacuation 63 et un conduit de sortie 64. Un filtre 65 est disposé au niveau du conduit de sortie 64, et est susceptible d'être dégradé par des caillots de sang se formant dans le circuit. Le piège à bulles veineux 6 comprend en outre une prise d'air 66, permettant l'entrée et la sortie d'air.
Le conduit de sortie 64 est relié à la ligne veineuse 7, tandis que l'orifice d'évacuation 63 est relié à la ligne de recirculation 8, qui est également reliée à la ligne artérielle 2 en amont de la pompe 3.

En présence de caillots dans le piège à bulles veineux 6, l'utilisateur rince le circuit avec du sérum puis obstrue la ligne veineuse 7, de manière à provoquer une montée du niveau de sérum dans le piège à bulles veineux 6 afin qu'il atteigne l'orifice d'évacuation 63.

Le sérum est ainsi dirigé vers la ligne de recirculation 8, ce qui permet de capter le caillot par exemple avec un piège à caillots 9 disposé dans la ligne de recirculation 8.
Une fois le caillot ainsi évacué du piège à bulles veineux 6, l'utilisateur cesse d'obstruer la ligne veineuse 7, et le sérum est remplacé par du sang, à nouveau acheminé vers le patient P, pour continuer la séance d'hémodialyse.

Toutefois, ce dispositif présente des inconvénients lors de son fonctionnement.
En effet, l'élévation du niveau de sérum dans le piège à bulles 6 entraîne également une montée du sérum dans les autres conduits débouchant en partie supérieure du piège à bulles, notamment dans l'orifice de pression 62 auquel est relié un capteur de pression, ce qui peut causer des dégradations importantes sur le circuit. En particulier, cela peut provoquer une contamination du générateur d'hémodialyse, ou fausser la lecture de la pression veineuse par le capteur de pression.
De plus, lors de l'acheminement de sang, ou plus généralement d'un fluide dans la ligne de recirculation 8, de l'air y est également entrainé et donc au travers de nombreux éléments du circuit 1, ce qui favorise la formation de nouveaux caillots.
La demande de brevet WO 2006/054957 au nom du demandeur en 2006 ne résout pas ces problèmes car elle ne permet pas de réussir le dégagement d'air du piège à bulle veineux sans que le fluide entre en contact direct avec l'orifice du conduit du capteur de pression.
On connaît par ailleurs du document US 5 591 251 A1 divers piège à bulles. Ces pièges à bulles comprennent des filtres empêchant des caillots d'être évacués hors de ces pièges.

### PRESENTATION DE L'INVENTION

La présente invention vise à proposer un dispositif ne présentant pas ces inconvénients.

En particulier, la présente invention vise à proposer un dispositif permettant de dégager l'air présent dans un piège à bulles sans que le fluide (sérum ou sang) n'entre en contact direct avec l'orifice de pression ou avec le capteur de pression.

A cet effet, l'invention propose un élément de piège à bulles d'un circuit extracorporel d'hémodialyse, comprenant
- un corps définissant un espace interne audit élément,
- une paroi interne divisant l'espace interne de l'élément en un premier et un second compartiment ;
- un orifice de pression adapté pour être relié à un capteur de pression afin de mesurer la pression au sein du piège à bulles ;
- un orifice d'évacuation pour l'évacuation de caillots se trouvant dans le piège à bulles ;
l'orifice de pression débouchant dans le premier compartiment de l'élément,
l'orifice d'évacuation débouchant dans le second compartiment de l'élément
caractérisé en ce que
ledit élément comprend en outre une prise d'air débouchant dans le second compartiment de l'élément.

Selon l'invention, l'élément du piège à bulles peut être réalisé au moyen d'un capuchon, fixé à une chambre de piège à bulles par des moyens de liaison, ou il peut s'agit d'un élément intégré à une chambre de piège à bulles, formant la partie supérieure de celle-ci.

Selon des modes de réalisation particuliers, l'élément de piège à bulles présente l'une ou plusieurs des caractéristiques suivantes, prises indépendamment ou en combinaison :
- le second compartiment présente une forme convergente vers l'orifice d'évacuation, typiquement en comprenant un conduit de forme sensiblement conique menant à l'orifice d'évacuation,
- le corps présente une paroi périphérique de section sensiblement circulaire et surmontée d'une cloison, et la paroi interne s'étend à partir de la cloison le long d'un diamètre de ladite paroi périphérique ;
- des conduits s'étendent dans le prolongement desdits orifices et de la prise d'air.

L'invention concerne par ailleurs un piège à bulles artériel ou veineux comprenant un élément tel que défini précédemment.

L'invention concerne également un circuit extracorporel d'hémodialyse muni d'un tel piège à bulles artériel ou veineux.

Selon un mode de réalisation particulier, ce circuit extracorporel d'hémodialyse comprend :
- une ligne artérielle sur laquelle sont successivement disposés une pompe, un piège à bulles artériel et un dialyseur et qui est reliée à un orifice d'alimentation du piège à bulles veineux ;
- une ligne veineuse, reliée à l'orifice de sortie du piège à bulles veineux,
- une ligne de recirculation munie d'un piège à caillots, reliée à l'orifice d'évacuation du piège à bulles veineux et à la ligne artérielle en amont de la pompe et à la ligne artérielle en amont de la pompe.

### PRESENTATION DES FIGURES

D'autres caractéristiques, buts et avantages de l'invention ressortiront de la description qui suit, qui est purement illustrative et non limitative, et qui doit être lue en regard des dessins annexés, sur lesquels :
- la figure 1 présentée précédemment illustre un circuit extracorporel d'hémodialyse selon l'état de la technique,
- la figure 2 présente un capuchon de piège à bulles selon un mode de réalisation de l'invention ;
- la figure 3a présente un tel capuchon associé à un piège à bulles veineux ;
- la figure 3b présente un élément de piège à bulles veineux sous forme d'élément intégré à la chambre d'un piège à bulles.
- la figure 4 illustre un circuit extracorporel d'hémodialyse muni d'un piège à bulles veineux selon un aspect de l'invention ;
- les figures 5a à 5e illustrent différentes étapes d'utilisation d'un piège à bulles veineux selon un aspect de l'invention,
- les figures 6 et 7 présentent deux vues d'un mode de réalisation de piège à bulles veineux selon un aspect de l'invention,
- la figure 8 présente un piège à bulles artériel selon un aspect de l'invention.

### DESCRIPTION DETAILLEE

Dans toute la suite on décrit un élément de piège à bulles selon un aspect de l'invention. Cet élément est de préférence un capuchon comme décrit ci-après, mais l'invention couvre également un élément de piège à bulles intégré dans un tel piège à bulles.

En outre, dans toute la suite, on pourra comprendre les termes « piège à bulles artériel » dans le sens de « chambre d'expansion artérielle (CEA) » et réciproquement.

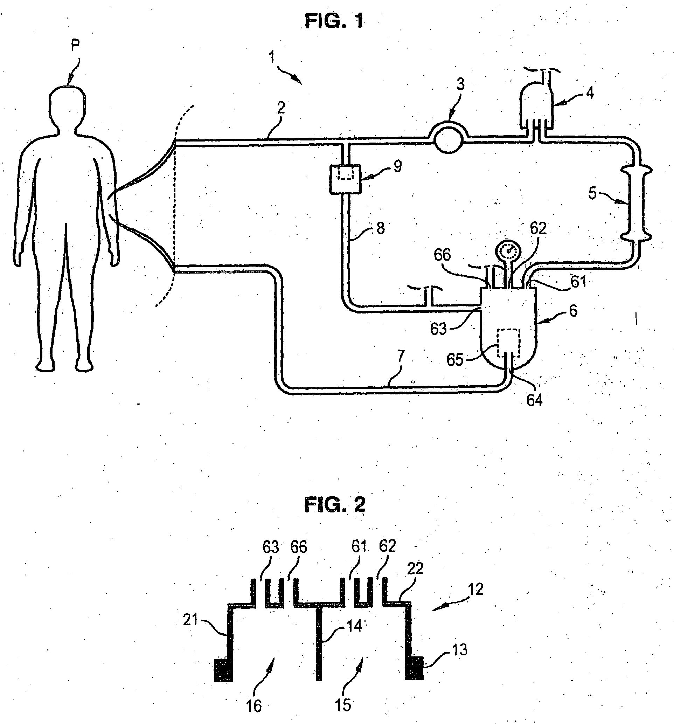

La figure 2 présente un capuchon de piège à bulles selon un aspect de l'invention.

Tel qu'illustré, le capuchon 12 comprend un corps formé d'une paroi périphérique 21 présentant une extrémité de liaison 13 munie de moyens de liaison et adaptée pour venir s'engager sur une extrémité correspondante de chambre de piège à bulles, et une extrémité obturée par une cloison 22.

Le corps du capuchon, et plus particulièrement la paroi périphérique 21 et la cloison 22 définissent ainsi un espace interne audit capuchon 12. Dans le mode de réalisation représenté, le capuchon 12 comprend un orifice d'alimentation 61, un orifice de pression 62, un orifice d'évacuation 63 et une prise d'air 66.

Le capuchon 12 est par ailleurs muni d'une paroi interne 14, divisant l'espace interne du capuchon 12 en deux compartiments 15 et 16. La paroi interne 14 s'étend typiquement à partir de la cloison 22 et sur toute la largeur du capuchon, par exemple diamétralement lorsque le capuchon 12 a une section circulaire.

L'orifice de pression 62 débouche dans le premier compartiment 15, et est adapté pour être relié à des moyens de mesure de pression, typiquement un manomètre.
L'orifice d'évacuation 63 débouche dans le second compartiment 16, et est adapté pour permettre l'évacuation de caillots de sang.
La prise d'air 66 débouche dans le second compartiment 16, et est adaptée pour permettre une admission ou une sortie d'air de l'espace interne du capuchon 12, plus particulièrement du second compartiment 16.
L'orifice d'alimentation 61 débouche dans le premier compartiment 15, et est typiquement obturé ou relié à une ligne d'alimentation en sang ou en sérum, selon que le capuchon 12 est associé à une chambre d'expansion artérielle ou à un piège à bulles veineux.
Dans le mode de réalisation représenté, l'orifice d'évacuation 63, la prise d'air 66, l'orifice de pression 62 et l'orifice d'alimentation 61 sont aménagés dans la cloison 22.

Les deux compartiments 15 et 16 du capuchon 12 sont séparés l'un de l'autre par la paroi 14, de sorte que les échanges de fluide ou de gaz entre ces deux compartiments contournent nécessairement la paroi 14 au niveau de son extrémité inférieure, opposée aux orifices 61, 62 et 63.

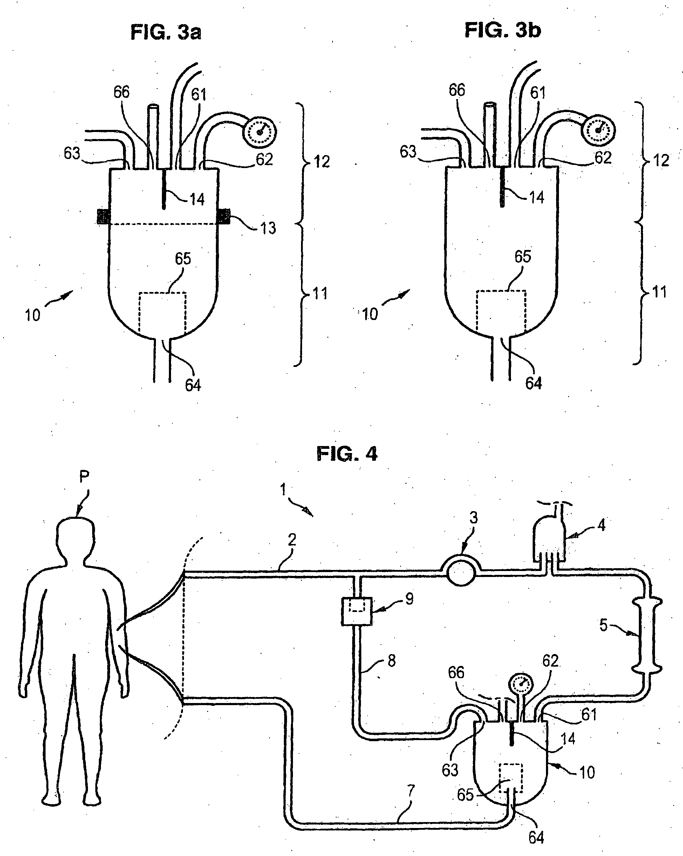

La figure 3a présente une vue du capuchon 12 associé à la chambre d'un piège à bulles veineux.
Le piège à bulles veineux 10 tel qu'illustré est ainsi composé d'une chambre 11 surmontée du capuchon 12.
La chambre 11 comprend un conduit de sortie 64 conduisant le sang vers la ligne veineuse, qui est surmonté d'un filtre 65.

Comme mentionné ci-avant, le piège à bulles veineux 10 peut être réalisé en une ou plusieurs parties ; l'élément 12 étant solidaire ou non de la chambre 11.

Un mode de réalisation dans lequel l'élément 12 est intégré à la chambre 11 du piège à bulles veineux est illustré en figure 3b, dans laquelle est représenté l'élément 12 en partie supérieure de la chambre 11 et, par exemple, venu de matière avec cette dernière. Dans ce mode de réalisation l'élément 12 de piège à bulles ne comporte alors pas de moyens de liaison avec la chambre 11.

Le piège à bulles veineux 10 est maintenu dans une position sensiblement verticale, de sorte que le conduit de sortie 64 soit en bas tandis que le capuchon 12 est en haut. Le sang acheminé dans le piège à bulles veineux 10 par l'orifice d'alimentation 61 tombe alors par gravité vers l'orifice de sortie 64.

En cas de présence d'un caillot de sang dans le piège à bulles veineux 10, l'utilisateur va rincer la circulation extracorporelle par du sérum, puis arrêter la pompe, obstruer le conduit de sortie 64 du piège à bulles veineux 10, ouvrir l'orifice de prise d'air 66, ce qui provoque une montée du niveau du sérum dans le piège à bulles veineux 10, l'air initialement présent dans le piège à bulles veineux 10 étant évacué par l'orifice de prise d'air 66.

Les deux compartiments 15 et 16 étant séparés par la paroi interne 14, une fois que le sérum a atteint le niveau de la paroi interne 14, l'air présent dans le premier compartiment 15 ne peut pas passer dans le second compartiment 16 afin de s'échapper via la prise d'air 66.
Ainsi, une fois que le niveau de sérum atteint la paroi interne 14, il cesse de monter dans le premier compartiment 15 dans la mesure où l'air qui y est présent ne peut pas s'en échapper, tandis que l'air présent dans le second compartiment 16 s'échappe par la prise d'air 66 et permet ainsi au niveau de sérum de continuer à monter dans le second compartiment 16 uniquement.

Le niveau de sérum dans le second compartiment 16 s'élève ainsi jusqu'à atteindre l'orifice d'évacuation 63, par lequel du sérum est mené à une voie de recirculation 8 similaire à celle présentée précédemment sur la figure 1, et le caillot est mené à un capteur de caillot disposé sur cette voie de recirculation 8.

La figure 4 illustre un circuit extracorporel d'hémodialyse 1 muni d'un piège à bulles veineux 10 selon un aspect de l'invention tel que présenté précédemment.

Le circuit 1 présenté sur cette figure comprend des éléments similaires à ceux présentés précédemment sur la figure 1, notamment :
- une ligne artérielle 2,
- une pompe 3,
- un piège à bulles artériel ou chambre d'expansion artérielle (CEA) 4,
- un dialyseur 5,
- un piège à bulles veineux 10,
- une ligne veineuse 7,
- une ligne de recirculation 8 et un piège à caillots 9.

Le sang est extrait du patient P via la ligne artérielle 2, et entrainé par la pompe 3 dans le circuit 1.
Le sang traverse ainsi la chambre d'expansion artérielle 4 (CEA), le dialyseur 5, le piège à bulles veineux 10, à partir duquel il est dirigé soit dans la ligne veineuse 7 pour être réinjecté dans le corps du patient P, soit dans la ligne de recirculation 8 pour circuler à nouveau dans le circuit 1.

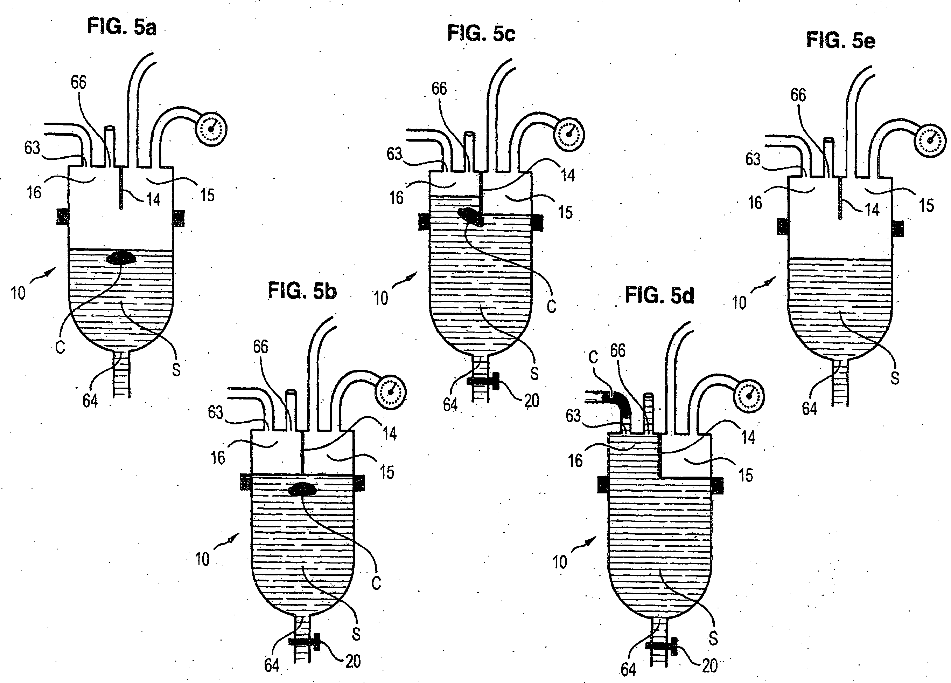

Les figures 5 illustrent plusieurs étapes de montée du niveau de sang S dans le piège à bulles veineux 10, et l'évacuation d'un caillot C de sang se trouvant dans le piège à bulles veineux 10 selon un aspect de l'invention.

La figure 5a illustre le piège à bulles veineux 10 dans lequel se trouve un volume de sang S et un caillot C formé dans ce volume de sang. En réponse à la présence de ce caillot C, l'utilisateur rince le circuit extracorporel par du sérum, arrête la pompe, obture le conduit de sortie 64 du piège à bulles veineux, typiquement au moyen d'un clamp 20, de manière à provoquer une montée du niveau de sérum S contenu dans le piège à bulles veineux 10. L'air présent dans le piège à bulles veineux 10 dont le sérum S vient prendre la place est évacué via la prise d'air 66.
Une fois que le niveau de sérum S a atteint la paroi interne 14, il reste à ce niveau dans le premier compartiment 15, et continue à monter uniquement dans le second compartiment 16 ; l'air contenu dans ce second compartiment 16 étant le seul qui est évacué via la prise d'air 66 qui débouche dans ce même second compartiment 16 comme illustré sur les figures 5b et 5c.
Le caillot C est alors évacué du piège à bulles veineux 10 via l'orifice d'évacuation 63, avec du sérum qui est typiquement acheminé vers la ligne de recirculation 8 afin d'être capté par le piège à caillots 9.
De cette manière; lorsque le caillot C est évacué du piège à bulles veineux 10, il n'y a plus d'air présent dans le second compartiment 16, et il n'y a donc pas d'air qui passe dans la ligne de recirculation 8. L'utilisateur cesse alors d'obturer le conduit de sortie 64 du piège à bulles veineux, et le volume de sang S baisse alors que de l'air est réintroduit dans le piège à bulles 10 veineux via la prise d'air 66.

Le piège à bulles veineux 10 tel que présenté, et plus précisément le capuchon 12 permet ainsi d'éviter que le fluide (sérum ou sang) ne remonte dans l'orifice de pression 62 et détériore des instruments de mesure. Il permet également de limiter la quantité d'air introduite dans le circuit extracorporel d'hémodialyse 1 via la ligne de recirculation 8 ce qui permet ainsi de réduire la formation de caillots dans le circuit 1.

Les étapes illustrées sur les figures 5a à 5e s'appliquent également dans le cas où le capuchon 12 est associé à une chambre d'expansion artérielle CEA 4, ou dans le cas où une telle chambre d'expansion artérielle présente une partie supérieure de même configuration que le capuchon 12.
Dans cette exploitation particulière, l'orifice d'alimentation 61 est supprimé ou obstrué, tandis que l'entrée de sang ou de sérum dans le piège à bulles ainsi que leur sortie s'effectue typiquement en partie basse de la chambre d'expansion artérielle CEA 4.

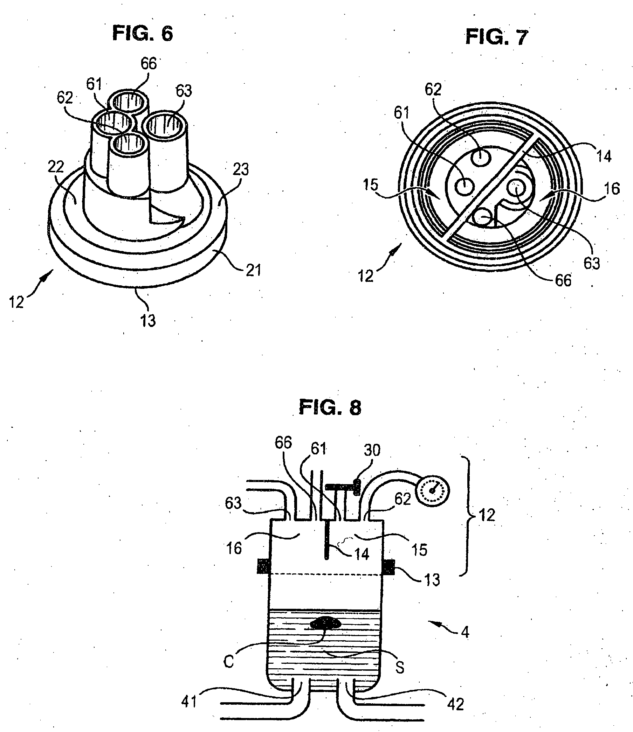

Les figures 6 et 7 présentent deux vues d'un mode de réalisation du capuchon 12.
La figure 6 représente une modélisation 3D du capuchon 12, tandis que la figure 7 présente une vue de dessous du capuchon 12, de manière à voir les compartiments 15 et 16.

Dans le mode de réalisation représenté, la paroi périphérique 21 est de forme sensiblement cylindrique de révolution, et reliée à la cloison 22 par un chanfrein 23.
Le capuchon 12 comprend des conduits s'étendant à partir de la cloison 22 dans le prolongement de l'orifice d'alimentation 61, l'orifice de pression 62, l'orifice d'évacuation 63 et de la prise d'air 66.
Les conduits ont typiquement une forme sensiblement cylindrique de révolution et des dimensions normalisées afin de permettre le raccordement des différentes lignes du circuit extracorporel d'hémodialyse 1 présentant des moyens de raccordement standardisés. Les orifices 61, 62, 61 et la prise d'air 66 tels que représentés ont une forme sensiblement cylindrique de révolution, d'axe parallèle à l'axe du capuchon 12.

La figure 7 illustre la répartition des orifices 61, 62, 63 et de la prise d'air 66 dans les deux compartiments 15 et 16 formés dans l'espace interne du couvercle 12 par la paroi interne 14 :
- l'orifice d'alimentation 61 et l'orifice de pression 62 débouchent dans le premier compartiment 15,
- l'orifice d'évacuation 63 et la prise d'air 66 débouchent dans le second compartiment 16.

Dans le mode de réalisation illustré, le second compartiment 16 du capuchon 12 présente une géométrie interne convergeant vers l'orifice d'évacuation 63, de manière à guider le caillot C dans ce conduit 63. Par exemple, le capuchon 12 peut présenter un conduit de forme sensiblement conique, menant du second compartiment 16 à l'orifice d'évacuation 63.

Dans le mode de réalisation illustré, la prise d'air 66 et l'orifice d'alimentation sont disposés sensiblement tangents à la paroi interne 14.
L'orifice d'évacuation est quant à lui légèrement espacé de la paroi interne 14, de manière à permettre la formation du conduit conique présenté précédemment.

L'orifice d'alimentation 61, l'orifice de pression 62, l'orifice d'évacuation 63 et la prise d'air 66 sont typiquement munis de moyens d'assemblage standards, permettant d'y associer des éléments du circuit d'hémodialyse.

Le capuchon 12 tel que présenté peut également être associé à un piège à bulles artériel 4, aussi appelé chambre d'expansion artérielle CEA.
La figure 8 illustre un exemple d'adaptation du capuchon 12 sur un piège à bulles artériel 4.

La chambre à bulles artérielle CEA 4 comprend une admission 41 et une sortie 42.

L'admission 41 est reliée à la ligne artérielle 2, et permet l'admission de sang dans le piège à bulles artériel 4. Le sang est ensuite évacué par la sortie 42 vers le dialyseur 5.
Dans le mode de réalisation représenté, l'admission 41 et la sortie 42 sont situées dans la partie inférieure de la chambre d'expansion artérielle CEA 4, c'est-à-dire la partie opposée au capuchon 12 une fois celui-ci positionné sur la chambre d'expansion artérielle 4.
Le capuchon 12 tel que présenté précédemment peut également être utilisé sur une telle chambre d'expansion artérielle CEA 4 pour l'évacuation de caillots de sang s'y trouvant.
En effet, de la même manière que pour le piège à bulles veineux, la partition du capuchon 12 en deux compartiments 15 et 16 permet de réaliser l'évacuation de caillots de sang par la partie supérieure du piège à bulles sans endommager des moyens de mesure de pression reliés à l'orifice de pression 62. Dans cette application particulière, l'orifice d'alimentation 61 du capuchon 12 est supprimé.

La capuchon 12 tel que présenté permet donc de réaliser une évacuation d'un caillot se trouvant dans un piège à bulles artériel CEA 4 ou veineux 10 d'un circuit extracorporel d'hémodialyse 1, en réalisant une montée du niveau du fluide (sang ou sérum) dans le piège à bulles 4 ou 10 tout en préservant les instruments de mesure de pression qui y sont présents grâce à la division du capuchon 12 en deux compartiments internes 15 et 16, et en ne permettant la montée du niveau de fluide (sang ou sérum) que dans l'un 16 de ces compartiments internes.

Selon un mode de réalisation avantageux, le capuchon 12 est réalisé en matériau transparent, permettant à l'utilisateur de détecter la présence de caillots présents dans le piège à bulles une fois le capuchon 12 mis en position. Ce caillot devient plus évident en rinçant le circuit extracorporel par 200 cc de sérum, ce qui permet son élimination.

Le capuchon 12 présente typiquement un diamètre compris entre 19 et 30 mm selon la marque du générateur d'hémodialyse utilisé.
La paroi interne 14 divisant l'espace interne du capuchon 12 a typiquement une hauteur comprise entre 5 et 15 mm.
La prise d'air 66, l'orifice de pression 62 et l'orifice d'alimentation 61 ont typiquement une section circulaire, et un diamètre compris entre 3 et 6 mm.
L'orifice d'évacuation 63 présente quant à lui typiquement une section circulaire, dont le diamètre est supérieur au diamètre des autres orifices 61, 62 et 66, par exemple compris entre 6 et 8 mm.

La description présentée précédemment concerne un circuit extracorporel d'hémodialyse 1 dans lequel circule du sang. On comprendra bien que le capuchon tel que présenté ne se limite pas à cette application, et permet d'évacuer de manière plus générale un corps se trouvant dans un fluide circulant dans un circuit extracorporel d'hémodialyse, typiquement un sérum.

## Revendications

1. Elément (12) de piège à bulles (4, 10) d'un circuit extracorporel d'hémodialyse (1), comprenant
- un corps définissant un espace interne audit élément (12),
- une paroi interne (14) divisant l'espace interne de l'élément (12) en un premier (15) et un second (16) compartiment ;
- un orifice de pression (62) adapté pour être relié à un capteur de pression afin de mesurer la pression au sein du piège à bulles (4, 10) ;
- un orifice d'évacuation (63) pour l'évacuation de caillots (C) se trouvant dans le piège à bulles (4, 10);
l'orifice de pression (62) débouchant dans le premier compartiment (15) de l'élément (12),
l'orifice d'évacuation (63) débouchant dans le second compartiment (16) de l'élément (12),
**caractérisé en ce que**
ledit élément (12) comprend en outre une prise d'air (66) débouchant dans le second compartiment (16) de l'élément (12).

2. Elément (12) selon la revendication 1, dans lequel le second compartiment (16) présente une forme convergente vers l'orifice d'évacuation (63).

3. Elément (12) selon la revendication précédente, dans lequel le second compartiment (16) présente un conduit de forme sensiblement conique menant à l'orifice d'évacuation (63).

4. Elément (12) selon l'une des revendications précédentes, dans lequel le corps présente une paroi périphérique (21) de section sensiblement circulaire surmontée d'une cloison (22), et la paroi interne (14) s'étend à partir de la cloison (22) le long d'un diamètre de ladite paroi périphérique (21).

5. Elément (12) selon la revendication précédente, dans lequel la paroi interne (14) a une hauteur comprise entre 5 et 15 mm.

6. Elément (12) selon l'une des revendications précédentes, comprenant des conduits s'étendant dans le prolongement desdits orifices (61, 62, 63) et de la prise d'air (66).

7. Elément (12) selon l'une des revendications précédentes, **caractérisé en ce qu'**il est réalisé en matériau transparent.

8. Piège à bulles artériel (4) ou veineux (10) comprenant un élément (12) selon l'une des revendications précédentes.

9. Circuit extracorporel d'hémodialyse (1) muni d'un piège à bulles artériel (4) ou veineux (10) selon la revendication précédente.

10. Circuit extracorporel d'hémodialyse (1) selon la revendication précédente, comprenant :
- une ligne artérielle (2) sur laquelle sont successivement disposés une pompe (3), un piège à bulles artériel (4) et un dialyseur (5) et qui est reliée un orifice, d'alimentation (61) du piège à bulles veineux (10) ;
- une ligne veineuse (7), reliée à l'orifice de sortie (64) du piège à bulles veineux (10),
- une ligne de recirculation (8) munie d'un piège à caillots (9), reliée à l'orifice d'évacuation (63) du piège à bulles veineux (10) et à la ligne artérielle (2) en amont de la pompe (3).

## Patentansprüche

1. Element (12) eines Blasenfängers (4, 10) eines extrakorporalen Hämodialysekreislaufs (1), Folgendes umfassend
- einen Korpus, der einen Innenraum für das besagte Element (12) definiert,
- eine Innenwand (14), welche den Innenraum des Elements (12) in ein erstes (15) und ein zweites (16) Fach unterteilt;
- eine Drucköffnung (62), die ausgeführt ist, um mit einem Drucksensor verbunden zu werden, um den Druck innerhalb des Blasenfängers (4, 10) zu messen;
- eine Evakuierungsöffnung (63) zum Evakuieren von Blutgerinnseln (C), die sich im Blasenfänger (4, 10) befinden;
wobei die Drucköffnung (62) in das erste Fach (15) des Elements (12) mündet,
wobei die Evakuierungsöffnung (63) in das zweite Fach (16) des Elements (12) mündet,
**dadurch gekennzeichnet, dass**
das besagte Element (12) darüber hinaus einen Lufteinlass (66) umfasst, der in das zweite Fach (16) des Elements (12) mündet.

2. Element (12) nach Anspruch 1, wobei das zweite Fach (16) eine konvergierende Form zur Evakuierungsöffnung (63) hin aufweist.

3. Element (12) nach dem vorhergehenden Anspruch, wobei das zweite Fach (16) einen Kanal in einer in etwa konischen Form aufweist, der zur Evakuierungsöffnung (63) führt.

4. Element (12) nach einem der vorhergehenden Ansprüche, wobei der Korpus eine umlaufende Wand (21) mit einem etwa kreisförmigen Querschnitt aufweist, der von einer Trennwand (22) überragt wird, und sich die Innenwand (14) von der Trennwand (22) entlang eines Durchmessers der besagten umlaufenden Wand (21) erstreckt.

5. Element (12) nach dem vorhergehenden Anspruch, wobei die Innenwand (14) eine Höhe aufweist, die zwischen 5 und 15 mm enthalten ist.

6. Element (12) nach einem der vorhergehenden Ansprüche, Kanäle umfassend, die sich in der Verlängerung der besagten Öffnungen (61, 62, 63) und des Lufteinlasses (66) erstrecken.

7. Element (12) nach einem der vorhergehenden Ansprüche, **dadurch gekennzeichnet, dass** es aus einem transparenten Material hergestellt ist.

8. Arterieller (4) oder venöser (10) Blasenfänger, ein Element (12) nach einem der vorhergehenden Ansprüche umfassend.

9. Extrakorporaler Hämodialysekreislauf (1), der mit einem arteriellen (4) oder venösen (10) Blasenfänger nach dem vorhergehenden Anspruch versehen ist.

10. Extrakorporaler Hämodialysekreislauf (1) nach dem vorhergehenden Anspruch, Folgendes umfassend:
- eine arterielle Leitung (2), in der nacheinander eine Pumpe (3), einen arteriellen Blasenfänger (4) und ein Dialysegerät (5) angeordnet sind, und die mit einer Zuführöffnung (61) zum venösen Blasenfänger (10) verbunden ist;
- eine venöse Leitung (7), die mit der Ausgangsöffnung (64) des venösen Blasenfängers (10) verbunden ist,
- eine Umwälzleitung (8), die mit einem Blutgerinnselfänger (9) versehen ist, der mit der Evakuierungsöffnung (63) des venösen Blasenfängers (10) und mit der arteriellen Leitung (2) stromaufwärts der Pumpe (3) verbunden ist.

## Claims

1. Bubble trap (4, 10) element (12) in an extracorporeal haemodialysis circuit (1) comprising
- a body defining a space internal to said element (12);
- an inner wall (14) dividing the inner space of the element (12) into a first (15) and a second (16) compartment;
- a pressure port (62) adapted to be connected to a pressure sensor in order to measure the pressure inside the bubble trap (4, 10);
- a discharge opening (63) to evacuate clots (C) located inside the bubble trap (4, 10);
the pressure port (62) opening up in the first compartment (15) of the element (12),
the discharge opening (63) opening up in the second compartment (16) of the element (12),
**characterised in that**
said element (12) also comprises an air intake (66) that opens up into the second compartment (16) of the element (12).

2. Element (12) according to claim 1, wherein the second compartment (16) has a geometry converging towards the discharge opening (63).

3. Element (12) according to the previous claim, wherein the second compartment (16) has an approximately conical shaped conduit leading to the discharge opening (63).

4. Element (12) according to one of the previous claims, wherein the body has a peripheral wall (21) with a substantially circular cross-section over which there is a partition (22), and the inner wall (14) extends from the partition (22) along a diameter of said peripheral wall (21).

5. Element (12) according to the previous claim, wherein the inner wall (14) is between 5 and 15 mm high.

6. Element (12) according to one of the previous claims, including conduits extending along said openings (61, 62, 63) and said air intake (66).

7. Element (12) according to one of the previous claims, **characterised in that** it is made from a transparent material.

8. Arterial (4) or venous (10) bubble trap comprising an element (12) according to one of the previous claims.

9. Extracorporeal haemodialysis circuit (1) provided with an arterial (4) or venous (10) bubble trap according to the previous claim.

10. Extracorporeal haemodialysis circuit (1) according to the previous claim, comprising:
- an arterial line (2) on which there is a pump (3), an arterial bubble trap (4) and a dialysis machine (5) in sequence and which is connected to a supply orifice (61) of the venous bubble trap (10) ;
- a venous line (7) connected to the outlet orifice (64) of the venous bubble trap (10),
- a recirculation line (8) provided with a clot trap (9), connected to the discharge opening (63) of the venous bubble trap (10) and to the arterial line (2) upstream of the pump (3).
